**Europäisches Patentamt**

**European Patent Office**

**Office européen des brevets**

(19)

(11) Veröffentlichungsnummer: **0 127 174**
**A2**

(12) ## EUROPÄISCHE PATENTANMELDUNG

(21) Anmeldenummer: **84106117.9**

(22) Anmeldetag: **29.05.84**

(51) Int. Cl.³: **A 61 M 37/00**

(30) Priorität: **31.05.83 DE 3319670**

(43) Veröffentlichungstag der Anmeldung:
**05.12.84 Patentblatt 84/49**

(84) Benannte Vertragsstaaten:
**AT BE CH DE FR GB IT LI LU NL SE**

(71) Anmelder: **LAPAG AG**
**Rorschacherstrasse 63**
**CH-9007 St. Gallen(CH)**

(72) Erfinder: **Latzke, Arno Walter**
**Oberlindenberg 191**
**CH-9427 Wolfhalden(CH)**

(74) Vertreter: **Werner, Hans-Karsten, Dr. et al,**
**Deichmannhaus am Hauptbahnhof**
**D-5000 Köln 1(DE)**

(54) **Mittel zur beschleunigten transdermalen Applikation von Wirkstoffen und Nährstoffen.**

(57) Die transdermale Applikation von Wirkstoffen und Nähr-stoffen wird beschleunigt durch permanent magnetisierbare und magnetisierte Kunststoffolien. Es werden Mittel und Vor-richtungen beschrieben, die diesen Effekt ausnutzen.

FIG.1

EP 0 127 174 A2

Gegenstand der vorliegenden Erfindung sind Mittel zur beschleunigten transdermalen Applikation von Wirkstoffen und Nährstoffen sowie ein Verfahren zur beschleunigten transdermalen Applikation von Wirkstoffen und Nährstoffen.

Wirkstoffe und Nährstoffe werden bisher noch in überwiegendem Maße oral oder in Form von Injektionen appliziert. Von geringerer Bedeutung ist bisher noch die an sich sehr alte percutane Applikation von Substanzen, mit denen sowohl ein lokale als auch eine allgemeine Wirkung erzielt werden kann. Zu diesem Zweck werden die Substanzen vorzugsweise in Form von Salben, Gelen und Lösungen auf die Haut aufgebracht und von dieser resorbiert. Die häufigste und von Natur aus bevorzugte Applikationsart ist zwar die orale, jedoch scheidet diese unter gewissen Umständen aus wegen Bewußtlosigkeit, Erkrankung, Überempfindlichkeit des Mundes, der Speiseröhre, des Magens oder des Darmtraktes sowie mangelnde Stabilität der Wirkstoffe gegenüber den Verdauungssäften. In diesen Fällen greift man bisher überwiegend zur Applikation durch Injektion, jedoch erfordert diese Applikationsart zusätzliche Geräte, einwandfrei sterilisierte Lösungen und entsprechend geschultes Personal. Außerdem ist die Injektion schmerzhaft und bei unsachgemäßer Durchführung gefährlich.

Es wird daher in der letzten Zeit in verstärktem Maße nach Wegen und Möglichkeiten gesucht, Substanzen percutan zu applizieren. Dabei hat man festgestellt, daß zwar eine Reihe von Substanzen durch die Haut aufgenommen werden können, jedoch die resorbierten Mengen pro Flächeneinheit in vielen Fällen viel zu gering sind, um dem Körper ausreichende und wirksame Mengen zuzuführen. In einigen Fällen ist es gelungen, durch spezielle Zubereitungen von Lösungen, Salben und Gelen die Resorption von Wirkstoffen erheblich zu erhöhen. Trotzdem besteht noch immer ein großes Bedürfnis, die transdermale Resorption von Substanzen zu beschleunigen und zu verstärken.

Neuere Untersuchungen über die Wirksamkeit und Anwendbarkeit von Magnetfeldern in der Therapie haben zu dem Ergebnis geführt, daß insbesondere magnetische Wechselfelder die Durchblutung der Haut erhöhen. Besonders wirksam sind nach diesen Untersuchungen die magnetischen Wechselfelder, die durch permanent magnetisierte Magnetfolien erzeugt werden. Derartige Magnetfolien sind daher in Form von selbstklebenden Pflastern oder mit Hilfe von hautverträglichen selbstklebenden Pflastern befestigte Folien in die Therapie eingeführt worden, vgl. P. Kokoschinegg, Engergy-Pak Mitteilungen für Ärzte und Apotheker 1, 1982, Therapeutische Magnetfolien und ihre Anwendung in der Humanmedizin. Weitere Untersuchungen haben jetzt ergeben, daß transdermal resorbierbare Wirkstoffe und Nährstoffe im Nahbereich von magnetischen Wechselfeldern in hohem Maße beschleunigt resorbiert werden. Der Grund für diese Erscheinung ist noch ungeklärt und kann sicherlich nicht allein auf die bessere Durchblutung der Haut im Nahbereich derartiger Magnetfolien zurückgeführt werden. Es erscheint vielmehr eine generelle Aktivierung der bioelektrischen Potentiale zu erfolgen, die sowohl die Aufnahme wie den Abtransport der transdermal resorbierten Substanzen beschleunigt. Diese Feststellungen eröffnen neue Möglichkeiten und Wege der transdermalen Applikation von transdermal resorbierbaren Wirkstoffen und Nährstoffen, da es erfindungsgemäß möglich ist, größere Mengen rasch zu resorbieren und dadurch die orale Applikation oder die Injektion zu vermeiden.

Gegenstand der vorliegenden Erfindung sind somit Mittel zur beschleunigten transdermalen Applikation von Wirkstoffen und Nährstoffen bestehend aus

a) einer wirksamen Menge eines transdermal resorbierbaren Wirkstoffes oder Nährstoffes in reiner Form oder in Form eines Gemisches mit Hilfs- und Trägerstoffen als Pulver, Tablette, Salbe, Gel, Lösung ggf. in einer Matrix oder einem für den Wirkstoff oder den Nährstoff durchlässigen Membran, und

b) einer 0,1 bis 8 mm starken, flexiblen, ggf. gelochten, gerillten oder mit sonstigen Aussparungen versehenen hautverträglichen, permanent magnetisierbaren und magnetisierten Kunststoffolie, welche selbstklebend ist, oder mit Hilfe von hautverträglichen selbstklebendem Pflaster oder einer Manschette auf der Haut zu befestigen ist.

Zu den transdermal resorbierbaren Wirkstoffen und Nährstoffen zählen all diejenigen niedermolekularen Substanzen, von denen schon heute bekannt ist, daß sie zu mindestens in geringen Mengen durch die Haut resorbiert werden können. Zu den Wirkstoffen zählen Analgetika, Antibiotika, Herz- und Kreislaufmittel, stoffwechselregulierende Substanzen, Hormone, Vitamine aber auch Nährstoffe wie Glucose, Aminosäuren und Mineralstoffe.

Die Wirkstoffe und Nährstoffe können sowohl in reiner Form als auch in Form eines Gemisches mit Hilfs- und Trägerstoffen aufgebracht werden, wobei prinzipiell alle üblichen Zubereitungsformen in Frage kommen, sofern aus diesen oberflächlich ausreichende Mengen der Substanzen freigesetzt werden. Außer Pulvern sind daher

- 4 -

Salben, Gele und Lösungen bevorzugt. Lösungen werden vorzugsweise in Form einer für den Wirkstoff oder Nährstoff durchlässigen Membran zugeführt, beispielsweise durch einen Schlauch-oder Rinnensystem mit kapillardurchlässiger Oberfläche. Die Membran kann auch in Form von Hohlfasern verschiedener Stärke vorliegen, wobei dickere Hohlfasern vorzugsweise in ein Rillensystem der Magnetfolie eingelagert werden sollten. In einigen Fällen kann es auch zweckmäßig sein, den Wirkstoff in einer Matrix einzulagern, die den Wirkstoff nur langsam abgibt, um dann durch die Magnetfelder die Resorption durch die Haut zu beschleunigen. Ein solches System würde eine sichere und gleichmäßige Zuführung des Wirkstoffes durch die Haut ermöglichen.

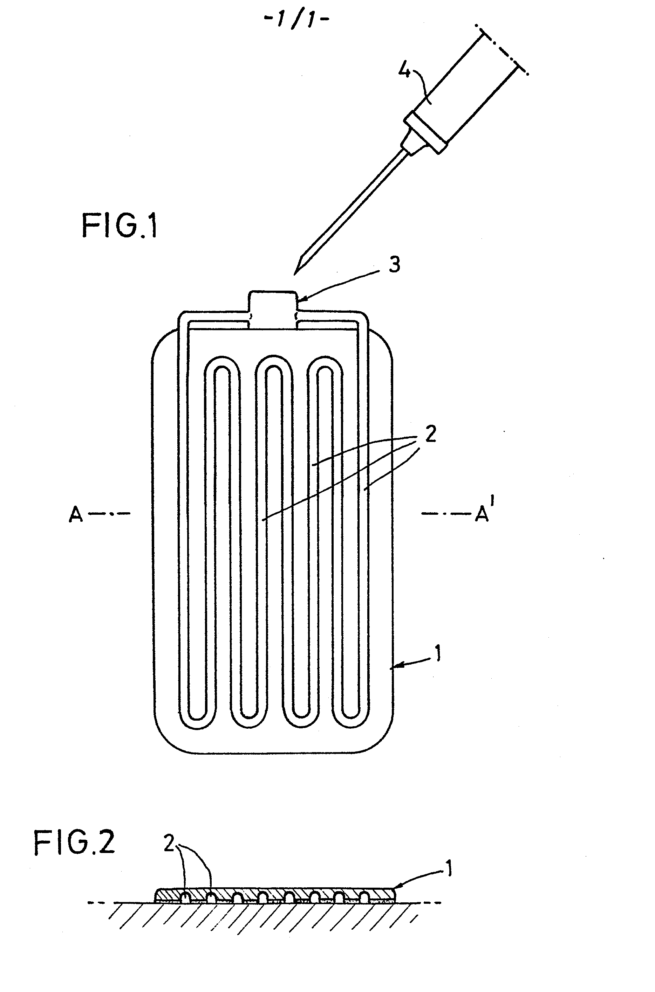

In den Figuren 1 und 2 ist schematisch eine Ausführungsform des erfindungsgemäßen Mittels beschrieben, bei der der Wirkstoff oder Nährstoff durch ein Schlauch- oder Rinnensystem mit kapillardurchlässiger Oberfläche zur Anwendung gebracht werden kann. In diesen Figuren bedeutet:

1) das selbstklebende Magnetpflaster;
2) ein Rinnensystem mit kapillardurchlässiger Oberfläche;
3) eine Vorratskammer;
4) eine Spritze.

Figur 2 zeigt einen Querschnitt auf der Schnittlinie A-A' im auf die Haut aufgeklebten Zustand.

Der Wirkstoff oder Nährstoff kann beispielsweise durch eine Spritze (4) in die freiliegende Vorratskammer (3) eingegeben werden und verteilt sich dann über das Rinnensystem mit Kapillargewebe (2) in den Bereich der Magnetfelder.

Die magnetischen Wechselfelder können zwar prinzipiell auf die verschiedenste Art und Weise erzeugt werden, für die praktische Anwendung sind jedoch hautverträgliche, permanent magnetisierbare und magnetisierte Kunststoffolien bevorzugt, die entweder selbstklebend sind oder mit Hilfe von hautverträglichen selbstklebenden Pflastern oder einer Manschette auf der Haut befestigt werden können.

Die Stärke der magnetisierten Kunststoffolien beträgt zwischen 0,1 und 8 mm. Bevorzugt werden Stärken zwischen 1,5 und 3 mm. Insbesondere wenn jedoch ein Rillensystem in die Magnetfolie eingelagert werden soll, empfiehlt es sich Stärken bis zu 8 mm zu verwenden.

Zur Anbringung und Unterbringung der Wirkstoffe oder Nährstoffe ist es zweckmäßig, die Magnetfolien zu lochen oder mit Rillen bzw. sonstigen Aussparungen zu versehen, in welchen die Wirkstoffe oder Nährstoffe untergebracht werden können. Im einfachsten Fall kann es genügen, den Wirkstoff in Form von Pulver auf die Haut aufzubringen und dieses Pulver mit Hilfe eines selbstklebenden Magnetpflasters mit Wechselfeldern auf der Haut zu befestigen. Insbesondere wenn größere Mengen an Wirkstoffen oder Nährstoffen über längere Zeit dem Körper zugeführt werden sollen, empfiehlt es sich Lösungen der Wirkstoffe oder Nährstoffe durch ein Membransystem hindurchzuleiten, welches in unmittelbaren Kontakt mit der Haut steht und durch ein Magnetpflaster auf der Haut festgehalten wird. Ggf. können derartige Membransysteme mit einem äußeren Vorratsbehälter verbunden sein und daher in ähnlicher Weise wie Dauertropfinfusionen zur künstlichen Ernährung und gleichzeitigen Applikation von Wirkstoffen eingesetzt werden.

Zur transdermalen Applikation von Wirkstoffen in kleinen Mengen können entsprechend klein dimensionierte Magnetpflaster mit einem Vorrat an Wirkstoff an beliebigen Stellen des Körpers aufgeklebt werden und ermöglichen so eine gleichmäßige und langanhaltende Applikation des Wirkstoffes in sehr bequemer Form. Diese Magnetpflaster können daher auch an solchen Stellen getragen werden, die weder optisch noch sonst als störend empfunden werden. Die gleichmäßige langanhaltende Zufuhr des Wirkstoffes wie beispielsweise Insulin kann zu einer wesentlich besseren Einstellung des Blutzuckerspiegels führen als die bisher noch notwendige Injektion von sofort wirksamen und Depot-Insulin. Weiterhin wird die lästige und mit gewissen Risiken verbundene Injektion vermieden.

Die erfindungsgemäßen Mittel können sowohl eingesetzt werden für Wirkstoffe die an gezielten Wirkungspunkten aufgebracht werden sollen oder auch solchen die den gesamten Organismus erreichen sollen. In einigen Fällen kann es besonders wertvoll sein, eine verstärkte Anflutung des Wirkstoffes an bestimmten Wirkungspunkten zu erreichen und gleichzeitig eine allmähliche Umverteilung des Wirkstoffes im gesamten Körper zu erreichen.

In den nachfolgenden Beispielen sind einige Ausführungsformen der erfindungsgemäßen Mittel näher beschrieben, jedoch ist es offensichtlich, daß es sich um ein völlig neues Prinzip handelt, welches generell und in sehr breiter Form zur Anwendung kommen kann.

# Beispiel 1

Handelsübliche Wechselfeldmagnetfolie mit einer Stärke von 1,5 mm und einem Abstand von streifenförmigen positiven und negativen Polen wurde in quadratische Stücke von ca. 2 x 2 cm zerschnitten. Zum Vergleich wurde die gleiche Folie jedoch im nicht magnetisierten Zustand verwendet. Kleine Mulläppchen wurden mit radioaktiv markierten Jod 125 getränkt und sowohl mit einfachem Heftpflaster, der nicht magnetisierten und der magnetisierten Folie auf die Haut von Mäusen aufgeklebt. Nach 32 Stunden wurden die Tiere getötet, enthäutet und liquefiziert. Die Radioaktivität des jeweils erhaltenen Breis wurde gemessen. Die Messergebnisse zeigten, daß die Radioaktivität der Gruppe mit der Wechselfeldmagnetfolie mindestens 3 mal so stark war wie die der beiden anderen Gruppen. Die beschleunigte Resorption des radioaktiven Jods war somit eindeutig auf die Magnetfelder in der Magnetfolie zurückzuführen.

P a t e n t a n s p r ü c h e

1.) Mittel zur beschleunigten transdermalen Applikation von Wirkstoffen und Nährstoffen bestehend aus

a) einer wirksamen Menge eines transdermal resorbierbaren Wirkstoffes oder Nährstoffes in reiner Form oder in Form eines Gemisches mit Hilfs- und Trägerstoffen als Pulver, Tablette, Salbe, Gel, Lösung ggf. in einer Matrix oder einem für den Wirkstoff oder den Nährstoff durchlässigen Membran, und

b) einer 0,1 bis 8 mm starken, flexiblen, ggf. gelochten, gerillten oder mit sonstigen Aussparungen versehenen hautverträglichen, permanent magnetisierbaren und magnetisierten Kunststoffolie, welche selbstklebend ist, oder mit Hilfe von hautverträglichen selbstklebendem Pflaster oder einer Manschette auf der Haut zu befestigen ist.

2.) Mittel gemäß Anspruch 1, dadurch gekennzeichnet, daß die Kunstfolie abwechselnd positiv und negativ magnetisiert ist und die positiven und negativen Bereiche einen Abstand von 1 bis 15 mm voneinander haben.

3.) Mittel gemäß Anspruch 2, dadurch gekennzeichnet, daß die Magnetfolie als abwechselnde parallele Streifen vorliegen.

4.) Mittel gemäß Anspruch 1 bis 3, dadurch gekennzeichnet, daß die Kunstfolie aus gummielastischem Material besteht.

5.) Verfahren zur beschleunigten transdermalen Applikation von Wirkstoffen und Nährstoffen, dadurch gekennzeichnet, daß man

a)     eine wirksame Menge eines transdermal resorbierbaren Wirkstoffes oder Nährstoffes in reiner Form oder in Form eines Gemisches mit Hilfs- und Trägerstoffen als Pulver, Tablette, Salbe, Gel, Lösung ggf. in einer Matrix oder einem für den Wirkstoff oder den Nährstoff durchlässigen Membran zusammen mit

b)     einer 0,1 bis 8 mm starken, flexiblen, ggf. gelochten, gerillten oder mit sonstigen Aussparungen versehenen hautverträglichen, permanent magnetisierbaren und magnetisierten Kunststoffolie, welche selbstklebend ist, oder mit Hilfe von hautverträglichen selbstklebendem Pflaster oder einer Manschette auf der Haut zu befestigen ist auf die Haut aufbringt.

0127174

FIG.1

FIG.2